# EUROPEAN PATENT SPECIFICATION

(11) **EP 3 813 640 B1**
(45) Date of publication and mention of the grant of the patent: **29.11.2023**
(21) Application number: 19732017.9
(22) Date of filing: 19.06.2019
(51) Int. Cl.: A61B 5/00, A61B 5/053

(54) **DEVICE FOR USE IN DETERMINING A HYDRATION AND/OR LIPID LEVEL OF SKIN**
VORRICHTUNG ZUR VERWENDUNG BEI DER BESTIMMUNG DES HYDRATISIERUNGSGRADES DER HAUT
DISPOSITIF DESTINÉ À ÊTRE UTILISÉ DANS LA DÉTERMINATION D'UN NIVEAU D'HYDRATATION DE LA PEAU

(30) Priority: 27.06.2018 EP 18180110
(43) Date of publication of application: 05.05.2021
(73) Proprietor: Koninklijke Philips N.V., 5656 AG Eindhoven (NL)
(72) Inventor: VARGHESE, Babu, 5656 AE Eindhoven (NL); SPOORENDONK, Wouter, Hendrik, Cornelis, 5656 AE Eindhoven (NL); BOURQUIN, Yannyk, Parulian, Julian, 5656 AE Eindhoven (NL); VAN DEN AKER, Karel, Johannes, Adrianus, 5656 AE Eindhoven (NL); VAN ABEELEN, Frank, Anton, 5656 AE Eindhoven (NL)
(74) Representative: Philips Intellectual Property & Standards
(86) International application number: PCT/EP2019/066190
(87) International publication number: WO 2020/002084

(56) References cited:
- EP-A1- 2 871 820
- EP-A1- 3 155 965
- WO-A1-03/094724
- WO-A1-2013/033724
- US-A1- 2011 144 525
- US-A1- 2015 002 168

## Description

### FIELD OF THE INVENTION

The disclosure relates to a device and a method of operating the device for use in determining a hydration and/or lipid level of skin.

### BACKGROUND OF THE INVENTION

A lipid (e.g. a skin surface lipid, such as sebum) level and a hydration (e.g. moisture or water) level of skin or, more specifically, the stratum corneum, are considered important factors in determining skin appearance and skin health. The right balance between these components is an indication of healthy skin and plays a central role in protecting and preserving skin integrity. An optimal balance between lipid and hydration levels provides the skin with a radiant, smooth texture and a natural pigmentation appearance. Excessive lipids on the skin can cause clogged pores possibly resulting in blemishes. Sufficient amount of skin hydration and lipids makes the skin appear smooth, soft and supple whereas lack of moisture can cause the skin to look dull and cracked, appearing older. The reduction in the efficiency of the barrier and moisture-maintaining functions of the skin result in easily dried, roughened skin, which can be potentially more vulnerable to risk of infection.

Several easy-to-use and high throughput in vivo non-invasive devices for skin hydration and lipid measurements exist that are well accepted by dermatologists. The most well-established commercially available existing devices for determining a hydration level of skin (e.g. the Corneometer^{®} CM 820, the Skicon^{®} 200, and the Nova DPM^{®} 9003) measure dielectric properties of skin, such as permittivity and conductivity. There also exist devices for measuring transepidermal water loss and capacitance for the assessment of skin water content and its water-holding capacity. The existing devices for determining a lipid level of skin include a sebumeter.

However, it is difficult for these existing devices to measure the hydration level and lipid level of the skin accurately, since the skin hydration measurements acquired by the existing devices are dependent on the amount of superficial lipids, such as sebum or oil, and the skin lipid measurements acquired by the existing devices are dependent on the skin hydration, such as moisture or water. The measurements acquired by existing devices are also dependent on other factors, such as the presence of sweat, the presence of hairs, the presence of artificial oil (e.g. moisturizing creams), the surface micro-topography, and environmental factors (e.g. humidity and temperature).

Thus, it is expected that the measurement of a hydration level and lipid level of skin obtained with existing devices can be affected by other factors. For example, lipids can have a very low value of dielectric constant compared to that of water. As such, if a thin layer of lipids is present in the probing depth of an existing device (which is typically in the range of few tens of microns), the hydration level of skin determined using such a device will naturally be impacted when the determination is based on the relatively high dielectric constant of water. Although a thin layer of lipids does not change the absolute baseline hydration level of skin directly, the skin hydration level measurements obtained by the existing devices are influenced due to the difference in the dielectric properties of lipids and water.

US 2008/0045816 discloses an apparatus for simultaneously measuring skin moisture content and a sweat glad activity to provide information to the user on both of these measurements. However, the skin moisture content measured by this apparatus is still affected by the presence of sweat, lipids and other factors. As such, this apparatus is also not capable of providing accurate measurements of the hydration level of skin. EP3155965 A1 discloses sub-epidermal moisture detection device using electrodes having different penetration depths.

Optical methods based on light absorption and/or scattering by specific molecules, such as Raman micro spectroscopy, are well known for their chemical specificity and high spatial resolution, which makes them inherently superior to traditional indirect electrical methods. In fact, confocal Raman micro spectroscopy is currently considered to be the gold standard for non-invasive quantitative, depth-resolved measurements of concentration profiles of molecular components through the skin, including water and lipids. However, these devices are expensive, complex and not affordable for consumer applications. Also, devices that use Raman microspectroscopy are not feasible for large area measurements (e.g. spatial mapping) and measurements using these devices also take a long time to acquire.

Thus, despite many technological developments throughout the years, there is still no low cost and easy to use device and method for the quantitative (and simultaneous) measurement of both skin superficial lipids and water.

### SUMMARY OF THE INVENTION

As noted above, a limitation with existing devices is that they provide inaccurate measurements of the hydration level and lipid level of skin due to the dependence of a hydration level measurement on the amount of lipids present on the surface of the skin and the dependence of a lipid level measurement on the hydration of (e.g. the amount of water or moisture present in) the skin. It would thus be valuable to have an improvement to address the existing problems.

Therefore, according to a first aspect, there is provided a device for use in determining a hydration and/or lipid level of skin. The device comprises at least two electrodes configured to provide an electrical signal to the skin and a spacer arranged such that the at least two electrodes provide the electrical signal to the skin at different penetration depths. The device also comprises a detector configured to measure a response received from the skin at the different penetration depths for use in determining the hydration and/or lipid level of the skin.

There is also provided a device for use in determining a hydration and/or lipid level of skin, which comprises a plurality of electrodes configured to provide an electrical signal to the skin and a spacer arranged to be positioned between at least one of the plurality of electrodes and the skin when the device is in use, such that the at least one of the plurality of electrodes and at least another one of the plurality electrodes are configured to provide the electrical signal to the skin at different penetration depths. The device also comprises a detector configured to measure a response received from the skin at the different penetration depths for use in determining the hydration and/or lipid level of the skin.

In some embodiments, the penetration depths may comprise any one or more of a penetration depth from an electrode of the at least two electrodes to a surface of the skin and a penetration depth from an electrode of the at least two electrodes to a location beneath the surface of the skin. According to the invention, the spacer is arranged to be positioned between at least one of the plurality of electrodes and the skin when the device is in use, such that the at least one of the plurality of electrodes is configured to provide the electrical signal to the skin at a first penetration depth from the at least one of the plurality of electrodes to a surface of the skin and the at least another one of the plurality electrodes is configured to provide the electrical signal to the skin at a second penetration depth from the at least another one of the plurality of electrodes to a location beneath the surface of the skin.

In some embodiments, the spacer may comprise a dielectric spacer. In some embodiments, the spacer may be patterned such that the at least two electrodes provide electrical signal to the skin at the different penetration depths. In some embodiments, a composition and/or thickness of the spacer may be selected based on a geometry of the at least two electrodes.

In some embodiments, the spacer may comprise a polyethylene terephthalate material, a polyelectric material, an aluminium material, or a polyetheretherketone material. In some embodiments, the spacer may be of a predefined thickness in a range from 1 micron to 100 microns. In some embodiments, the spacer may be arranged such that the spacer is positioned between one or more of the at least two electrodes and the skin in use.

In some embodiments, the at least two electrodes may comprise at least two pixelated electrodes.

In some embodiments, the device may further comprise a signal generator configured to generate the electrical signal and provide the electrical signal to the at least two electrodes.

According to a second aspect, there is provided a system for determining a hydration level and/or lipid level of skin. The system comprises a device as described earlier and a processor. The processor is configured to acquire, from the detector, the measured response received from the skin at the different penetration depths and determine the hydration level and/or lipid level of the skin based on the measured response received from the skin at the different penetration depths.

In some embodiments, the processor may be configured to classify the determined hydration level and/or lipid level of the skin into a class based on a comparison of the response received from the skin at the different penetration depths.

According to a third aspect, there is provided a method of operating a device for use in determining a hydration level and/or lipid of skin. The device comprises at least two electrodes, a spacer, and a detector. The method comprises providing, by the at least two electrodes, an electrical signal to the skin. The spacer is arranged such that the at least two electrodes provide the electrical signal to the skin at different penetration depths. The method also comprises measuring, by the detector, a response received from the skin at the different penetration depths for use in determining the hydration level and/or lipid level of the skin.

There is also provided a method of operating a device for use in determining a hydration level and/or lipid level of skin. The device comprises a plurality of electrodes, a spacer, and a detector. The method comprises providing, by the plurality of electrodes, an electrical signal to the skin. The spacer is arranged to be positioned between at least one of the plurality of electrodes and the skin when the device is in use, such that the at least one of the plurality of electrodes and at least another one of the plurality electrodes provide the electrical signal to the skin at different penetration depths. The method also comprises measuring, by the detector, a response received from the skin at the different penetration depths for use in determining the hydration level and/or lipid level of the skin.

In some embodiments, the method may further comprise generating, by a signal generator, the electrical signal and providing, by the signal generator, the electrical signal to the at least two electrodes

In some embodiments, the method may further comprise acquiring, by a processor from the detector, the measured response received from the skin at the different penetration depths and determining, by the processor, the hydration level of the skin based on the measured response received from the skin at the different penetration depths.

According to a fourth aspect, there is provided a computer program product comprising a computer readable medium, the computer readable medium having a computer readable code embodied therein, the computer readable code being configured such that, on execution by a suitable computer or processor, the computer or processor is caused to perform the method described above.

According to the aspects and embodiments described above, the limitations of existing techniques are addressed. In particular, according to the above-described aspects and embodiments, it is possible to determine a hydration level and/or a lipid level of skin more accurately. This is possible since the confounding influence of lipids on the hydration level measurements and/or the confounding influence of hydration (e.g. moisture or water) on the lipid level is minimized by the spacer of the device enabling the skin response to be measured at different penetration depths for use in determining the hydration level of the skin. As different penetration depths can be achieved, it is possible to measure a response received from the skin at different penetration depths for use in determining the hydration and/or lipid level of the skin. It is thus possible to non-invasively measure a hydration level and/or a lipid level of skin (e.g. and even simultaneously measure the hydration level and lipid level of skin) in view of the manner in which the spacer is arranged to allow different penetration depths for measurements. There is thus provided an improved device and method of operating the device for determining a hydration and/or lipid level of skin, which is aimed at overcoming existing problems.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a block diagram of a device according to an embodiment;
Fig. 2 illustrates example spacers according to embodiments;
Fig. 3 is flow chart illustrating a method according to an embodiment;
Fig. 4 illustrates skin hydration measurements obtained by an existing device;
Fig. 5 illustrates skin hydration measurements obtained by an existing device;
Fig. 6 illustrates skin hydration measurements obtained using a skin response measured by a device according to an embodiment;
Fig. 7 illustrates skin hydration measurements obtained using a skin response measured by a device according to another embodiment;
Fig. 8 illustrates a comparison of skin hydration measurements obtained using a skin response measured by a device according to an embodiment and an existing device;
Fig. 9 illustrates skin lipid measurements obtained using an existing device;
Fig. 10 illustrates skin lipid measurements obtained using a skin response measured by a device according to an embodiment;
Fig. 11 illustrates a plot used to classify hydration and lipid levels of skin;
Fig. 12 illustrates a plot used to classify skin types based on hydration and lipid levels of skin; and
Fig. 13 illustrates a relationship of skin conditions to hydration and lipid levels of skin.

### DETAILED DESCRIPTION OF EMBODIMENTS

As noted above, there is provided herein an improved device and method of operating the device for determining a hydration (e.g. moisture or water) level and/or a lipid (e.g. sebum) level of skin. In some embodiments, the device described herein can be a device for treating skin and/or for diagnosing one more skin conditions. In other embodiments, the device described herein can be a separate (e.g. a stand-alone) device for use with a device for treating skin and/or for diagnosing one more skin conditions. The device described herein can be for determining a hydration level and/or lipid level of skin from any part of the body of a subject, such as a finger (e.g. the device may be a finger print device), a thumb, a face, or any other part of the body of the subject.

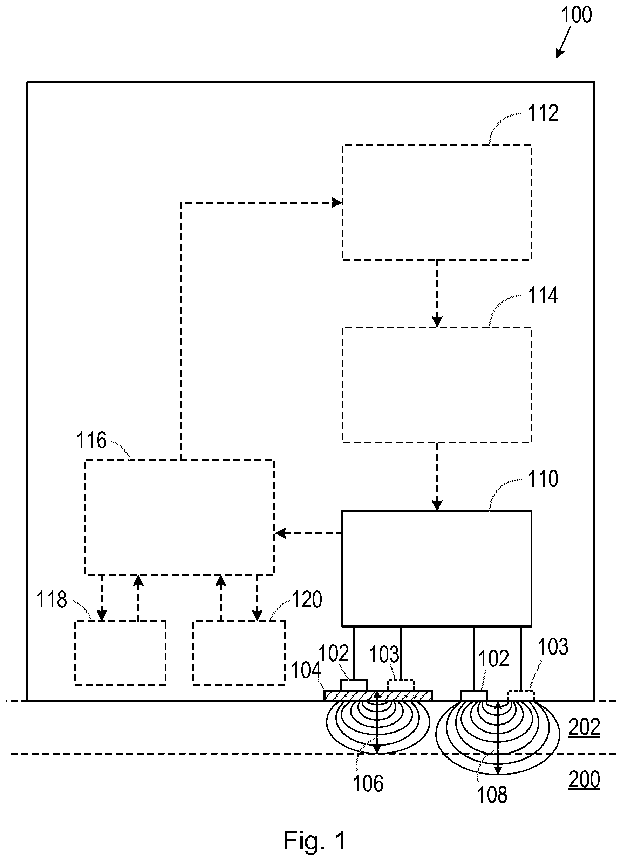

Fig. 1 illustrates a device 100 for use in determining a hydration level and/or lipid level of skin 200. As illustrated in Fig. 1, the device 100 comprises at least two (i.e. a plurality of, e.g. an array of) electrodes 102 configured to provide an electrical signal to the skin 200. As illustrated in Fig. 1, the device 100 may also comprise at least two (i.e. a plurality of, e.g. an array of) further electrodes 103, each further electrode configured to receive the electrical signal provided to the skin 200 by one of the at least two electrodes 102. In some embodiments, the at least two electrodes 102 may comprise at least two (i.e. a plurality of, e.g. an array of) active electrodes 102 configured to transmit the electrical signal and the at least two further electrodes 103 may comprise at least two (i.e. a plurality of, e.g. an array of) return electrodes 103 configured to receive the electrical signal transmitted from a corresponding one of the at least two active electrodes 102. Thus, in some embodiments, the device 100 can comprise at least two pairs of electrodes or an array of electrodes. The at least two pairs of electrodes or the array of electrodes can comprise the at least two active electrodes 102 and at least two corresponding return electrodes 103.

The device 100 is for use on skin 200, which may comprise a lipid layer 202. The skin 200 can comprise moisture, e.g. water. In embodiments where the device 100 comprises at least two active electrodes 102 and at least two return electrodes 103, the electrical signal can be provided across each active electrode 102 and corresponding return electrode 103. In use, each active electrode 102, corresponding return electrode 103 and a lipid layer 202 on the skin 200 (if present) or the skin 200 and the lipid layer 202 on the skin 200 (if present) form an electrical circuit. Thus, an electrical signal provided across an active electrode 102 and a corresponding return electrode 103 passes through a lipid layer 202 on the skin 200 (if present) or the skin 200 and the lipid layer 202 on the skin 200 (if present) between the active electrode 102 and corresponding return electrode 103. As illustrated in Fig. 1, the electrical signal can comprise a plurality of electrical field lines between each active electrode 102 and corresponding return electrode 103.

In some embodiments, any one or more of the at least two active electrodes 102 and the at least two return electrodes 103 can comprise a microelectrode. An active electrode of the at least two active electrodes 102 may be located at any suitable distance from a return electrode of the at least two return electrodes 103. For example, in some embodiments, an active electrode of the at least two active electrodes 102 may be located at a distance in a range from 0.1 mm to 1 mm from a return electrode of the at least two return electrodes 103. In this way, the sensitivity of the device 100 may be increased. In some embodiments, the at least two electrodes 102 configured to provide an electrical signal to the skin 200 may comprise at least two pixelated electrodes.

A person skilled in the art will be familiar with electrodes that are suitable for providing an electrical signal to skin 200 and also electrodes that are suitable for receiving an electrical signal from the skin 200. A person skilled in the art will also be familiar with an arrangement and/or a geometry for the at least two active electrodes 102 that is suitable for providing an electrical signal to skin 200 and also for the at least two return electrodes 103 that is suitable for receiving an electrical signal from the skin 200. For example, the at least two active electrodes 102 and/or the at least two return electrodes 103 may be provided in an interlaced arrangement, a multi-pin arrangement, a concentric arrangement, a skin chip arrangement, or any other arrangement of which the skilled person will be aware.

As illustrated in Fig. 1, the device 100 also comprises a spacer (or a spacer material) 104. The spacer 104 is arranged such that the at least two electrodes 102 provide the electrical signal to the skin 200 at different penetration (or probing) depths 106, 108. That is, the spacer 104 is arranged such that the plurality of electrodes 102 provide the electrical signal to the skin 200 at different penetration depths 106, 108. In more detail, the spacer 104 can be arranged to be positioned between at least one of the plurality of electrodes 102 and the skin 200 when the device 100 is in use, such that the at least one of the plurality of electrodes 102 and at least another one of the plurality electrodes 102 are configured to provide the electrical signal to the skin 200 at the different penetration depths 106, 108. That is, as illustrated in Fig. 1, at least one of the plurality of electrodes 102 can be provided with the spacer 104 and at least another one of the plurality electrodes 102 can be provided without the spacer 104, such that the at least one of the plurality of electrodes 102 and at least another one of the plurality electrodes 102 are configured to provide the electrical signal to the skin 200 at the different penetration depths 106, 108.

The penetration depths 106, 108 can comprise a first penetration depth 106 and a second penetration depth 108, wherein the first penetration depth 106 is different to the second penetration depth 108. For example, as illustrated in Fig. 1, the first penetration depth 106 may be less than the second penetration depth 108. In effect, the spacer 104 reduces the penetration depth. Thus, the depth 106 to which the electrical signal provided by the at least one of the plurality of electrodes 102 with the spacer 104 penetrates is reduced compared to the depth 108 to which the electrical signal provided by the at least another one of the plurality of electrodes 102 without the spacer.

In some embodiments, the penetration depths 106, 108 may comprise a first penetration depth 106 that is confined to a lipid (e.g. sebum) layer 202 on the skin 200 (if present). For example, the penetration depths 106, 108 may comprise a first penetration depth 106 that is (e.g. a distance) from an electrode of the at least two electrodes 102 to a surface of the skin 200. In these embodiments, as illustrated in Fig. 1, where the electrical signal comprises a plurality of electrical field lines between each active electrode 102 and corresponding return electrode 103, the electrical field lines can be confined to the lipid layer 202 on the skin 200 (if present). Alternatively, or in addition, in some embodiments, the penetration depths 106, 108 may comprise a second penetration depth 108 that is (e.g. a distance) from an electrode of the at least two electrodes 102 to a location beneath the surface of the skin 200. In these embodiments, as illustrated in Fig. 1, where the electrical signal comprises a plurality of electrical field lines between each active electrode 102 and corresponding return electrode 103, the electrical field lines are not confined to the lipid layer 202 on the skin 200 but also extend beneath the surface of the skin 200.

In some embodiments, the different penetration depths 106, 108 can comprise a first penetration depth 106 of up to 10 microns, for example up to 9 microns, for example up to 8 microns, for example up to 7 microns, for example up to 6 microns, for example up to 5 microns, for example up to 4 microns, for example up to 3 microns, for example up to 2 microns, for example up to 1 micron and a second penetration depth 108 of up to 40 microns, for example up to 35 microns, for example up to 30 microns, for example up to 25 microns, for example up to 20 microns.

In some embodiments, as illustrated in Fig. 1, the spacer 104 can be arranged such that the spacer 104 is positioned between one or more of the at least two electrodes 102 that are configured to provide an electrical signal to the skin 200 and the skin 200 in use. The spacer 104 can be arranged to at least partially cover a surface of the device 100 that is configured to contact the skin 200 in use. The surface of the device 100 can be configured to contact the skin 200 directly or indirectly in use, such as via a lipid layer 202. In some embodiments, the spacer 104 can be patterned (or segmented). The spacer 104 can be patterned such that the at least two electrodes provide the electrical signal to the skin 200 at the different penetration depths 106, 108. For example, the spacer 104 may be patterned such that, in use, at least part of the spacer 104 is positioned between one or more of the at least two electrodes 102 and the skin 200. In some embodiments, the spacer 104 may be patterned such that, in use, at least part of the spacer 104 is positioned between alternate ones of the at least two electrodes 102 and the skin 200. In effect, one or more of the at least two electrodes 102 may be provided with a spacer 104, while the remainder of the at least two electrodes 102 may be provided without a spacer 104.

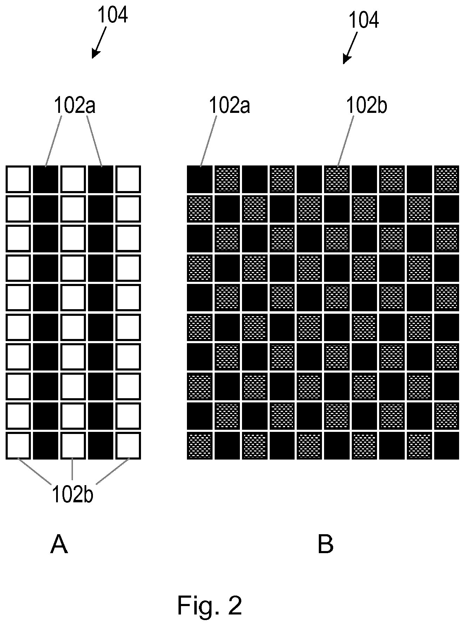

Fig. 2 illustrates example spacers 104 according to some embodiments, where the spacer 104 is patterned such that the at least two electrodes provide the electrical signal to the skin 200 at the different penetration depths 106, 108. In the embodiment illustrated in Fig. 2A, the spacer 104 is patterned such that, in use, at least part of the spacer 104 is positioned between the skin 200 and each alternate row of the at least two electrodes 102. That is, alternate rows of the at least two electrodes 102 may be provided with the spacer 104 according to some embodiments. Thus, in effect, in the embodiment illustrated in Fig. 2A, a plurality of the at least two electrodes 102 are provided with the spacer 104 and a plurality of the at least two electrodes are provided without the spacer 104. In Fig. 2A, the areas provided with the spacer 104 are illustrated by the black squares 102a and the areas provided without the spacer 104 are illustrated by the white squares 102b. In some embodiments, each of the areas 102a, 102b may comprise at least two active electrodes 102. According to some embodiments, the distance between these at least two active electrodes 102 and their size can define the penetration depth 106, 108, with the penetration depth 106 of the at least two electrodes 102 provided with the spacer 104 being confined to a shallower depth than the penetration depth 108 of the at least two electrodes provided without the spacer 104.

In the embodiment illustrated in Fig. 2B, the spacer 104 is patterned such that, in use, at least part of the spacer 104 is positioned between alternate ones of the at least two electrodes 102 and the skin 200. That is, alternate ones of the at least two electrodes 102 are provided with the spacer 104. Thus, in effect, in the embodiment illustrated in Fig. 2B, a plurality of the at least two electrodes 102 are provided with the spacer 104 and a plurality of the at least two electrodes 102 are provided without the spacer 104. In Fig. 2B, the areas provided with the spacer 104 are illustrated by the black squares 102a and the areas provided without the spacer 104 are illustrated by the grey squares 102b. In some embodiments, each of the areas 102a, 102b may comprise at least two active electrodes 102. According to some embodiments, the distance between these at least two active electrodes 102 and their size can define the penetration depth 106, 108, with the penetration depth 106 of the at least two electrodes 102 provided with the spacer 104 being confined to a shallower depth than the penetration depth 108 of the at least two electrodes provided without the spacer 104.

In some embodiments, the spacer 104 may comprise a material that is at least partially insulating and at least partially conducting, such that at least some electrical signals pass through the spacer 104 to the skin 200. In some embodiments, the spacer 104 may comprise a dielectric spacer, e.g. a spacer 104 made of a dielectric material. In some embodiments, the spacer 104 may comprise a polyethylene terephthalate (PET) material, a polyelectric (PZE) material, an aluminium material, or a polyetheretherketone (PEEK) material. In some embodiments, the spacer 104 may comprise a foil.

The spacer 104 can have a predefined composition or a tunable (or adjustable) composition. The predefined composition of the spacer 104 can, for example, comprise one or more predefined dielectric properties. The tunable composition of the spacer 104 can, for example, comprise one or more tunable dielectric properties. For example, the spacer 104 may be a tunable dielectric material (such as a piezoelectric material). Alternatively or in addition to the composition of the spacer 104, the spacer 104 may have a predefined thickness. The composition and/or the thickness of the spacer 104 can be used to tune (or adjust) the first penetration depth 106.

In some embodiments, a composition (or type) of the spacer 104 may be selected based on a geometry of the at least two electrodes 102, the size of the at least two electrodes 102 and/or the distance between the at least two electrodes 102 (e.g. the inter-electrode distances). Alternatively, or in addition, in some embodiments, a thickness of the spacer 104 may be selected based on the geometry of the at least two electrodes 102, the size of the at least two electrodes 102 and/or the distance between the at least two electrodes 102 (e.g. the inter-electrode distances). For example, in some embodiments, the larger the at least two electrodes 102 of the device 100 and/or the larger the distance between the at least two electrodes 102 of the device 100, the thicker the selected spacer 104 for the device 100. That is, for example, a device 100 comprising large electrodes 102 with long distances between them may comprise a thicker spacer 104 than a device 100 comprising thin electrodes 102 with short distances between them.

Thus, in some embodiments, the composition and/or thickness of the spacer 104 may be different for different electrode geometries, different size electrodes and/or different distances between electrodes. In embodiments where the composition and/or thickness of the spacer 104 is selected, the composition and/or thickness of the spacer 104 may define the different penetration depths 106, 108. In some embodiments, the spacer 104 may be of a predefined thickness. The predefined thickness may, for example, be of the order of few microns to tens of microns. For example, in some embodiments, the spacer 104 may be of a predefined thickness in a range from 1 micron to 100 microns.

In some embodiments, the spacer 104 can be removable from the device 100. That is, it may be possible to remove the spacer 104 from the device 100. In this way, different spacers 104 may be used, e.g. for different applications. For example, spacers 104 having different compositions and/or different predefined thicknesses may be used.

Returning back to Fig. 1, as illustrated, the device 100 further comprises a detector 110. The detector 110 is configured to measure a response received from the skin 200 at the different penetration depths 106, 108 for use in determining the hydration level and/or lipid level of the skin 200. In effect, the spacer 104 can enable the detector 110 to acquire dual measurements with a spacer 104 and without a spacer 104. The detector 110 may be configured to measure the response received from the skin 200 at the different penetration depths 106, 108 via at least two return electrodes 103. As different penetration depths 106, 108 can be achieved in the manner described herein, it is possible for the detector 110 to measure a response received from the skin 200 at different penetration depths 106, 108 for use in determining the hydration and/or lipid level of the skin 200.

In some embodiments, the detector 110 can be configured to measure the response received from the skin 200 at the different penetration depths 106, 108 by being configured to measure a voltage across each active electrode 102 and corresponding return electrode 103. Alternatively, or in addition, in some embodiments, the detector 110 can be configured to measure the response received from the skin 200 at the different penetration depths 106, 108 by being configured to measure a current along the circuit formed by each active electrode 102, corresponding return electrode 103 and a lipid layer 202 on the skin 200 (if present) or the skin 200 and the lipid layer 202 on the skin 200 (if present). Alternatively, or in addition, in some embodiments, the detector 110 can be configured to measure the response received from the skin 200 at the different penetration depths 106, 108 by being configured to measure an impedance of the circuit formed by each active electrode 102, corresponding return electrode 103 and a lipid layer 202 on the skin 200 (if present) or the skin 200 and the lipid layer 202 on the skin 200 (if present). In the latter case, the detector 110 may comprise one or more impedance sensors (e.g. a single impedance sensor or an array of impedance sensors) or an impedance measurement system.

As illustrated in Fig. 1, in some embodiments, the spacer 104 can be arranged to be positioned between at least one of the plurality of return electrodes 103 and the skin 200 when the device 100 is in use (e.g. in the same way as the spacer 104 is arranged to be positioned between at least one of the plurality of active electrodes 102 and the skin 200 when the device 100 is in use, as described earlier). That is, as illustrated in Fig. 1, at least one of the plurality of return electrodes 103 can be provided with the spacer 104 and at least another one of the plurality of return electrodes 103 can be provided without the spacer 104. In more detail, the spacer 104 can be arranged to be positioned between at least one of the plurality of active electrodes 102 and the skin 200 and also between at least one of the plurality of corresponding return electrodes 103 and the skin 200 when the device 100 is in use.

As illustrated in Fig. 1, in some embodiments, the device 100 may comprise a signal generator 112. However, it will be understood that, in other embodiments, the signal generator 112 may be external to (i.e. separate to or remote from) the device 100. For example, in some embodiments, the signal generator 112 can be a separate entity or part of another device. The signal generator 112 can be configured to generate the electrical signal. The signal generator 112 can also be configured to provide the electrical signal to the at least two electrodes 102. For example, the signal generator 112 can be configured to provide the electrical signal across each active electrode 102 and corresponding return electrode 103.

In some embodiments, the signal generator 112 can be configured to generate the electrical signal at a certain (e.g. predefined) frequency. In some embodiments, the signal generator 112 may be configured to generate frequency pulses. The frequency pulses may, for example, be fixed frequency pulses or variable frequency pulses. In some embodiments, the pulses may comprise low-voltage pulses. In some embodiments, the electrical signal may comprise a radio frequency (RF) signal. Thus, in some embodiments, the signal generator 112 can be configured to generate a radio frequency (RF) signal. In some of these embodiments, where the signal generator 112 is configured to generate frequency pulses, the frequency pulses may comprise radio frequency pulses.

As illustrated in Fig. 1, in some embodiments, the device 100 may comprise an amplifier 114, such as a radio frequency (RF) amplifier. In these embodiments, the signal generator 112 can be configured to provide the electrical signal to the at least two electrodes 102 via the amplifier 114. The amplifier 114 can be configured to amplify the electrical signal provided by (or a voltage of an output of) the signal generator 112.

As illustrated in Fig. 1, in some embodiments, the device 100 may comprise a processor 116. However, it will be understood that, in other embodiments, the processor 116 may be external to (i.e. separate to or remote from) the device 100. For example, in some embodiments, the processor 116 can be a separate entity or part of another device. The processor 116 may also be referred to as a control system.

In some embodiments, the processor 116 may be configured to control the at least two electrodes 102 to provide the electrical signal to the skin 200. In some embodiments, the processor 116 may be configured to control the at least two electrodes 102 to control any one or more of a frequency, a voltage and a pulse duration of the electrical signal that the at least two electrodes 102 are configured to provide to the skin 200. In some embodiments, the processor 116 may be configured to control the detector 110 to measure the response received from the skin 200 at the different penetration depths 106, 108. In some embodiments, the processor 116 can be configured to acquire, from the detector 110, the measured response received from the skin 200 at the different penetration depths 106, 108. For example, the output of the detector 110 can be provided to (e.g. fed into) the processor 116 according to some embodiments.

In some embodiments, the processor 116 can be configured to determine the hydration level and/or lipid level of the skin 200 based on the measured response received from the skin 200 at the different penetration depths 106, 108. For example, in some embodiments, the processor 116 may be configured to determine the hydration level and/or lipid level of the skin 200 by being configured to process the measured response received from the skin 200 at the different penetration depths 106, 108 to determine a measure of a permittivity at the different penetration depths 106, 108 (ε1, ε2). Thus, in effect, the processor 116 can be configured to measure the permittivity with the spacer 104 (ε1) and without the spacer 104 (ε2). The lipid level and/or hydration level of the skin 200 can be derived from the permittivity measured with the spacer 104 (ε1) and without the spacer 104 (ε2), i.e. the permittivity measured at the different penetration depths 106, 108 (ε1, ε2). In particular, the lipid level of the skin 200 (or, more specifically, the lipid level of the lipid layer 202 on the skin 200) can be derived from the permittivity measured with the spacer 104 (ε1), i.e. at the first penetration depth 106, and the hydration level of the skin 200 can be derived from the permittivity measured without the spacer 104 (ε2), i.e. at the second penetration depth 108. In some embodiments, the processor 116 can be configured to determine the hydration and/or lipid level of the skin 200 based on the measured response received from the skin 200 at the different penetration depths 106, 108 by way of a ratio of the measure of permittivity at the different penetration depths 106, 108 (ε1, ε2), e.g. the hydration level of the skin 200 may be determined as (ε2- ε1)/ (ε2* ε1). However, a person skilled in the art will be aware of various ways in which a hydration level and/or lipid level may be derived from a measured permittivity.

In some embodiments, the processor 116 can be configured to apply an offset correction to correct for the contribution of the spacer 104 to the permittivity measured with the spacer 104 (ε1). This provides a net permittivity (ε3) excluding the offset correction. The offset correction can, for example, be defined based on the type and/or thickness of the spacer 104. The lipid level and/or hydration level of the skin 200 can be derived from the permittivity measured with the spacer 104 (ε1) and the net permittivity (ε3) excluding the offset correction. In particular, the lipid level of the skin 200 (or, more specifically, the lipid level of the lipid layer 202 on the skin 200) can be derived from the permittivity measured with the spacer 104 (ε1), i.e. at the first penetration depth 106 (ε1), and the hydration level of the skin 200 can be derived from the net permittivity (ε3) excluding the offset correction (ε3), i.e. at the second penetration depth 108. In some embodiments where an offset correction is applied, the processor 116 can be configured to determine the hydration level and/or lipid level of the skin 200 based on the measured response received from the skin 200 at the different penetration depths 106, 108 by way of a ratio of the corrected measure of permittivity at the different penetration depths 106, 108 (ε1, ε3), e.g. the hydration level of the skin 200 may be determined as (ε3- ε1)/ (ε3* ε1). However, as mentioned earlier, a person skilled in the art will be aware of various ways in which a hydration level and/or lipid level may be derived from a measured permittivity.

Also, a person skilled in the art will be aware of various methods by which a permittivity may be measured from a response received from the skin 200. Alternatively or in addition, in some embodiments, the processor 116 can be configured to determine the hydration level and/or lipid level of the skin 200 using image analysis and/or machine learning. For example, the processor 116 may be configured to acquire an image of the skin 200 with the spacer 104 and an image of the skin 200 without the spacer 104, i.e. at the different penetration depths 106, 108. In these embodiments, the processor 116 may be configured to use image analytics and/or a machine learnt model to process the images to determine the hydration level and/or lipid level of the skin 200.

Although examples have been provided for the way in which the processor 116 may be configured to determine the hydration level and/or lipid level of the skin 200 based on the measured response received from the skin 200 at the different penetration depths 106, 108, it will be understood that alternative ways (e.g. alternative permittivity ratios) are also possible and a person skilled in the art will be aware of such alternative ways in which the processor 116 may be configured to determine the hydration level and/or lipid level of the skin 200 based on the measured response received from the skin 200 at the different penetration depths 106, 108.

Generally, in embodiments comprising a processor 116, the processor 116 can be configured to control the operation of the device 100 to implement the method described herein. In some embodiments, the processor 116 may comprise one or more processors. The one or more processors can be implemented in numerous ways, with software and/or hardware, to perform the various functions described herein. In some embodiments, each of the one or more processors can be configured to perform individual or multiple steps of the method described herein. In particular implementations, the one or more processors can comprise a plurality of software and/or hardware modules, each configured to perform, or that are for performing, individual or multiple steps of the method described herein. The one or more processors may comprise one or more microprocessors, one or more multi-core processors and/or one or more digital signal processors (DSPs), one or more processing units, and/or one or more controllers (such as one or more microcontrollers) that may be configured or programmed (e.g. using software or computer program code) to perform the various functions described herein.

In some implementations, for example, the processor 116 may comprise a plurality of (for example, interoperated) processors, processing units and/or modules, multi-core processors and/or controllers configured for distributed processing. It will be appreciated that such processors, processing units and/or modules, multi-core processors and/or controllers may be located in different locations and may perform different steps and/or different parts of a single step of the method described herein. The one or more processors may be implemented as a combination of dedicated hardware (e.g. amplifiers, pre-amplifiers, analog-to-digital convertors (ADCs) and/or digital-to-analog convertors (DACs)) to perform some functions and one or more processors (e.g. one or more programmed microprocessors, DSPs and associated circuitry) to perform other functions.

As illustrated in Fig. 1, in some embodiments, the device 100 may comprise a memory 118. Alternatively, or in addition, the memory 118 may be external to (e.g. separate to or remote from) the device 100. The processor 116 may be configured to communicate with and/or connect to the memory 118. The memory 118 may comprise any type of non-transitory machine-readable medium, such as cache or system memory including volatile and non-volatile computer memory such as random-access memory (RAM), static RAM (SRAM), dynamic RAM (DRAM), read-only memory (ROM), programmable ROM (PROM), erasable PROM (EPROM), and electrically erasable PROM (EEPROM). In some embodiments, the memory 118 can be configured to store program code that can be executed by the processor 116 to cause the device 100 to operate in the manner described herein. Alternatively, or in addition, in some embodiments, the memory 118 can be configured to store information required by or resulting from the method described herein. For example, in some embodiments, the memory 118 may be configured to store any one or more of the response received from the skin 200 at the different penetration depths 106, 108, a hydration level and/or lipid level of the skin 200 that may be determined using the received response, or any other information, or any combination of information, required by or resulting from the method described herein. In some embodiments, the processor 116 can be configured to control the memory 118 to store information required by or resulting from the method described herein.

As illustrated in Fig. 1, in some embodiments, the device 100 may comprise a user interface 120. Alternatively, or in addition, the user interface 120 may be external to (e.g. separate to or remote from) the device 100. The processor 116 may be configured to communicate with and/or connect to a user interface 120. The user interface 120 can be configured to render (e.g. output, display, or provide) information required by or resulting from the method described herein. For example, in some embodiments, the user interface 120 may be configured to render (e.g. output, display, or provide) any one or more of the response received from the skin 200 at the different penetration depths 106, 108, a hydration level and/or lipid level of the skin 200 that may be determined using the received response, or any other information or any other information, or any combination of information, required by or resulting from the method described herein. Alternatively, or in addition, the user interface 120 can be configured to receive a user input. For example, the user interface 120 may allow a user to manually enter information or instructions, interact with and/or control the device 100. Thus, the user interface 120 may be any user interface that enables the rendering (or outputting, displaying, or providing) of information and, alternatively or in addition, enables a user to provide a user input.

For example, the user interface 120 may comprise one or more switches, one or more buttons, a keypad, a keyboard, a mouse, a touch screen or an application (e.g. on a smart device such as a tablet, a smartphone, smartwatch, or any other smart device), a display or display screen, a graphical user interface (GUI) such as a touch screen, or any other visual component, one or more speakers, one or more microphones or any other audio component, one or more lights (e.g. light emitting diode LED lights), a component for providing tactile or haptic feedback (e.g. a vibration function, or any other tactile feedback component), a smart device (e.g. a smart mirror, a tablet, a smart phone, a smart watch, or any other smart device), or any other user interface, or combination of user interfaces. In some embodiments, the user interface that is controlled to render information may be the same user interface as that which enables the user to provide a user input. In some embodiments, the processor 116 can be configured to control the user interface 120 to operate in the manner described herein.

Although not illustrated in Fig. 1, in some embodiments, the device 100 may comprise a communications interface (or communications circuitry). Alternatively, or in addition, the communications interface may be external to (e.g. separate to or remote from) the device 100. The communications interface can be for enabling the device 100, or components of the device 100, to communicate with and/or connect to one or more other components, sensors, interfaces, devices, or memories (such as any of those described herein). For example, the communications interface can be for enabling any one or more of the earlier described at least two (active) electrodes 102, at least two return electrodes 103, detector 110, signal generator 112, amplifier 114, processor 116, memory 118, and user interface 120 to communicate with and/or connect to each other. The communications interface may enable the device 100, or components of the device 100, to communicate and/or connect in any suitable way. For example, the communications interface may enable the device 100, or components of the device 100, to communicate and/or connect wirelessly, via a wired connection, or via any other communication (or data transfer) mechanism. In some wireless embodiments, for example, the communications interface may enable the device 100, or components of the device 100, to use radio frequency (RF), Bluetooth, or any other wireless communication technology to communicate and/or connect.

Although also not illustrated in Fig 1, the device 100 may comprise a battery or other power supply for powering the device 100 or means for connecting the device 100 to a mains power supply. It will be understood that Fig. 1 only shows the components required to illustrate an aspect and, in a practical implementation, the device 100 may comprise any other component to those described herein or any combination of components.

In addition to the device 100, there is also provided a system for determining a hydration level and/or lipid level of skin 200. The system comprises the device 100 described earlier for use in determining a hydration level and/or lipid level of skin 200. The system can also comprise any one or more of the earlier described at least two return electrodes 103, signal generator 112, amplifier 114, processor 116, memory 118, and user interface 120.

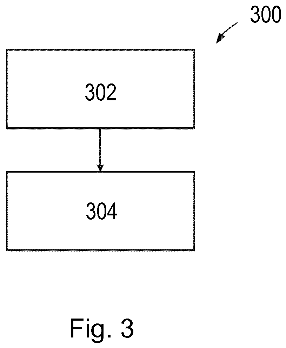

Fig. 3 illustrates a method 300 of operating the device 100 described earlier for use in determining a hydration level and/or lipid level of skin 200. As described earlier, the device 100 comprises at least two electrodes 102, a spacer 104 and a detector 110. In some embodiments, the method 300 illustrated in Fig. 3 can be performed under the control of the processor 116 described earlier. At block 302 of Fig. 3, an electrical signal is provided to the skin 200. More specifically, the electrical signal is provided by the at least two electrodes 102. As described earlier, the spacer 104 is arranged such that the at least two electrodes 102 provide the electrical signal to the skin 200 at different penetration depths 106, 108. At block 304 of Fig. 3, a response received from the skin 200 at the different penetration depths 106, 108 for use in determining the hydration level and/or lipid level of the skin 200 is measured. More specifically, the response is measured by the detector 110.

Although not illustrated in Fig. 3, in some embodiments, the method 300 may comprise generating the electrical signal and providing the electrical signal to the at least two electrodes 102. More specifically, the method 300 may comprise the electrical signal being generated and provided by the signal generator 112. Although also not illustrated in Fig. 3, in some embodiments, the method 300 may comprise acquiring the measured response received from the skin 200 at the different penetration depths 106, 108 and determining the hydration level and/or lipid level of the skin 200 based on the measured response received from the skin 200 at the different penetration depths 106, 108. More specifically, the method 300 may comprise the measured response being acquired by the processor 116 from the detector 110 and the processor 116 determining the hydration level and/or lipid level of the skin 200. In some embodiments, the method 300 may comprise relaying the determined hydration level and/or lipid level of the skin 200 (e.g. when the determined hydration level and/or lipid level of the skin 200 is outside a predefined range according to some embodiments) to the user interface 120. More specifically, the method 300 may comprise the determined hydration level and/or lipid level of the skin 200 being relayed by the processor 116 to the user interface 120. It will be understood that the method 300 may comprise any other steps, and any combination of steps, corresponding to the operation of the device 100 described earlier with reference to Figs. 1 and 2.

As mentioned earlier, the device 100 and method 300 described herein enables a hydration level of skin 200 to be determined more accurately. This is possible since the confounding influence of lipids on the hydration level measurements is minimized by the spacer 104 of the device 100 enabling the skin 200 response to be measured at different penetration depths for use in determining the hydration level of the skin 200. Although a thin layer of lipids does not change the absolute baseline hydration level of skin directly, the skin hydration level measurements obtained by the existing devices are influenced due to the difference in the dielectric properties of lipids and water.

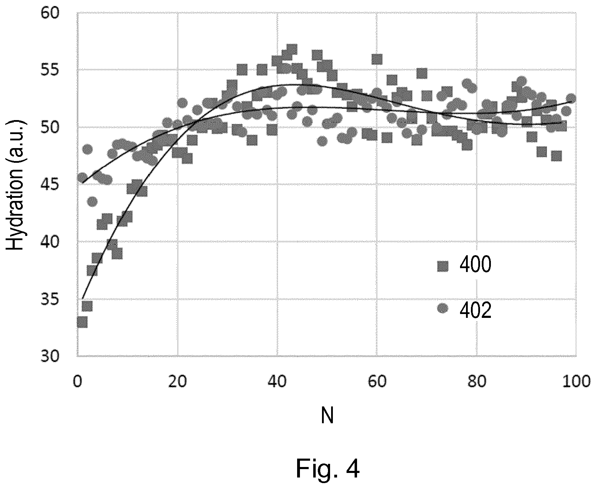

This can be observed in Fig. 4, which illustrates skin hydration measurements obtained from a Corneometer and normalized to the maximum value. The skin hydration measurements are based on skin capacitance and are obtained from the T-zone of forehead (illustrated by the points 400) and the forearm (illustrated by the points 402), which are known to have different levels of sebum content. In particular, the T-zone of forehead has a high level of sebum content, whereas the forearm has a low level of sebum content. In Fig. 4, the normalized skin hydration measurements (on the y-axis) are plotted against the number of measurements N taken at the same location (on the x-axis).

It can be seen from Fig. 4 that measurements repeated on the same location (N=100) showed that the hydration measurements gradually increase. This is due to the local occlusion when a probe of the device is in contact with the skin and due to the reduction in the amount of sebum after each measurement. In particular, each measurement removes some sebum, which means that successive measurements lead to a reduction in sebum and thus an increase in the hydration measurements. The increase is more pronounced on the forehead and, in particular, during the initial measurements when sebum is consistently present before it is gradually removed after each subsequent measurement. Thus, it can be seen from Fig. 4 that the existing methods used for skin hydration measurements can be influenced by the quantity of lipids on the surface of the skin.

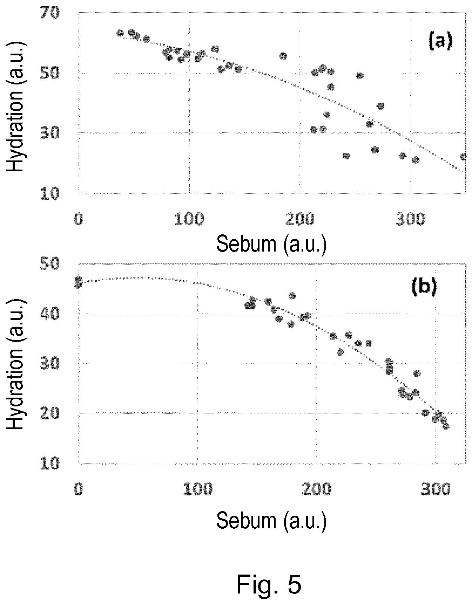

This can also be observed in Fig. 5, which illustrates a skin hydration level measured with a Corneometer normalized to the maximum value (on the y-axis) and plotted as a function of sebum level measured with a Sebumeter (on the x-axis). As can be seen from Fig. 5, the skin hydration measured with the Corneometer decreases as the sebum level increases, even though the absolute skin hydration is not changing.

In more detail, Fig. 5(a) shows an increase in hydration level as the use of the Sebumeter on the skin removes sebum for each measurement that is obtained. In this example, the hydration level is kept constant at a baseline level of 45 arbitrary units (a.u.) while the sebum is gradually removed. As illustrated in Fig. 5(a), the sebum levels drop from 300 arbitrary units (a.u.) to approximately 50 arbitrary units (a.u.) as the corresponding hydration levels increase from 20 to 60 arbitrary units (a.u.). Fig. 5(b) shows a decrease in hydration level as an amount of oil is increased by applying increasing amounts of artificial sebum on the skin. The hydration level decreases by a factor of two, from 40 to 20 arbitrary units (a.u.) when a thin layer of sebum is applied to the skin. In this example, the hydration level is kept constant at a baseline level of 45 arbitrary units (a.u.) while sebum concentration is increased by adding artificial sebum up to a final concentration of 1.2 µl/cm², which corresponds to a sebum level of 300 arbitrary units (a.u.).

Thus, as illustrated in Fig. 5, the hydration level depends strongly on the amount of lipids present at the surface of the skin. This insight into the dependency of the hydration level on the amount of lipids present at the surface of the skin has led to the device 100 and method 300 described herein, which overcomes the limitations associated with the dependency to enable a hydration level of skin 200 to be determined more accurately.

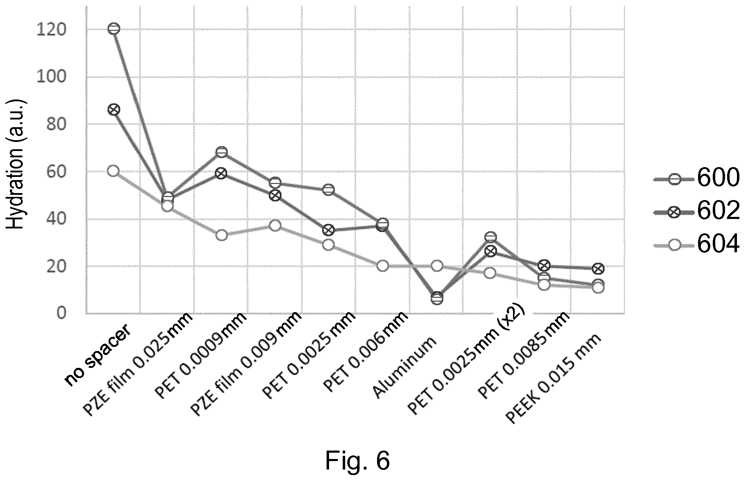

Fig. 6 illustrates the effect of using the device 100 described herein comprising different spacers 104 to measure a response received from the skin 200 at the different penetration depths 106, 108 for use in determining the hydration level of the skin 200. In more detail, Fig. 6 illustrates skin hydration measurements derived from the response measured using the device 100 and normalized to the maximum value (on the y-axis). The normalized skin hydration measurements are plotted as a function of material type and thickness for the spacer 104 (on the x-axis). For comparison purposes, Fig. 6 also illustrates skin hydration measurements obtained from an existing device with no spacer. The skin hydration measurements are provided in arbitrary units (a.u.) and the thickness is provided in millimeters (mm) in Fig. 6. The skin hydration measurements are obtained from a calibration pad soaked with liquids of different permittivity, namely Corneometer calibration (CK) solution 600, Ethyleen Glycol 602, and 1-Butanol 604.

A decrease in the skin hydration measurements can be seen in Fig. 6. This decrease is related to a decreasing penetration depth and the physical properties of the materials used for the spacer 104, such as the thickness and permittivity of the spacer 104. A factor related to the material used for the spacer 104 can be determined and compensated by a correction factor. If the observed decrease in the skin hydration measurements were only related to the presence of the spacer 104, the skin hydration measurements would not depend on the permittivity of the liquids in which the calibration pad is soaked as they do in Fig. 6. Thus, as illustrated in Fig. 6, it can be beneficial to confine the penetration depth 106 to superficial layers at or on the surface of the skin 200 by using the spacer 104 described herein. Furthermore, it can be seen from Fig. 6 that the confinement of the penetration depth 106 is different for different materials and thickness of spacer 104.

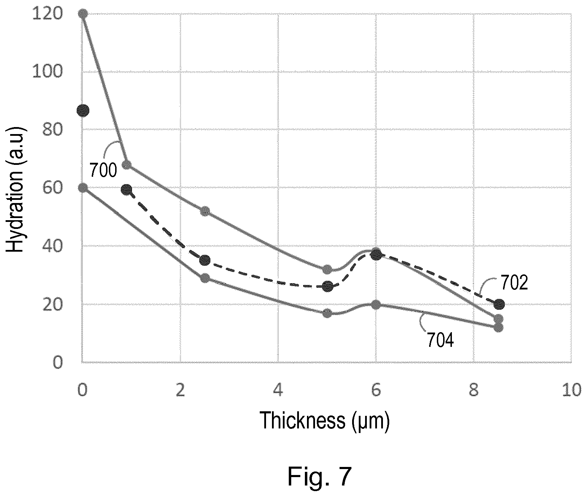

Fig. 7 illustrates the effect of using the device 100 comprising a spacer 104 as described herein to measure a response received from the skin 200 at the different penetration depths 106, 108 for use in determining the hydration level of the skin 200. In this illustrated example, the spacer 104 comprises a polyethylene terephthalate (PET) foil. In more detail, Fig. 7 illustrates skin hydration measurements derived from the response measured using the device 100 and normalized to the maximum value (on the y-axis). The normalized skin hydration measurements are plotted as a function of the thickness of the spacer 104 (on the x-axis). The skin hydration measurements are provided in arbitrary units (a.u.) and the thickness is provided in micrometers (µm) in Fig. 7. The skin hydration measurements are obtained from a calibration pad soaked with liquids of different permittivity, namely a Corneometer calibration (CK) solution 700, Ethyleen Glycol 702, and 1-Butanol 704.

As illustrated in Fig. 7, it can be beneficial to confine the penetration depth 106 to superficial layers at or on the surface of the skin 200 by using the spacer 104 described herein. Furthermore, it can be seen from Fig. 7 that the confinement of the penetration depth 106 depends on the thickness of spacer 104. It can be seen that, the thicker the spacer 104, the larger the confinement of the penetration depth using the spacer 104. For completeness, it is noted that some of the thickness measurements illustrated are a combination of two spacers 104 on top of each other. When this is the case (e.g. at 5 and 8.5 µm), air between the spacers 104 lowers the actual hydration level measurements compared to a single layer spacer (e.g. at 6 µm) where no air is present.

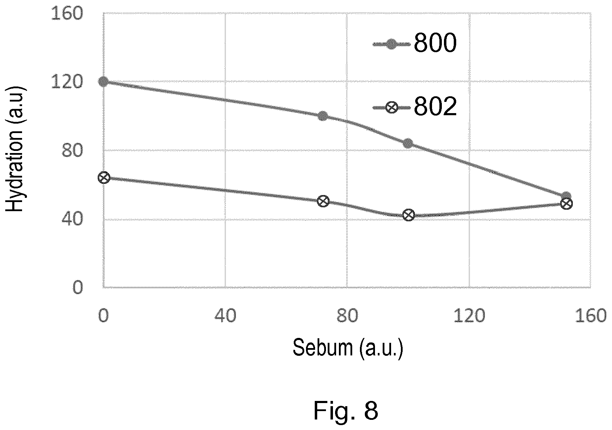

Fig. 8 illustrates a comparison of the influence of an amount (or level) of sebum on skin hydration measurements obtained using an existing device without a spacer (illustrated by the line labelled 800) and skin hydration measurements derived from the response measured using the device 100 comprising the spacer 104 described herein (illustrated by the line labelled 802). In more detail, Fig. 8 illustrates the skin hydration measurements normalized to the maximum value (on the y-axis) as a function of the amount of sebum (on the x-axis). The skin hydration measurements and the amount of sebum are provided in arbitrary units (a.u.) in Fig. 8. The skin hydration measurements derived from the response measured using the device 100 comprising the spacer 104 are shown after correction for the contribution of the spacer 104. In this example, the spacer 104 comprises a polyethylene terephthalate (PET) foil with a thickness of 3 µm.

The skin hydration measurements are obtained from a calibration pad soaked with water and with different levels of sebum placed on the top surface of the pad. The sebum level is varied from 0-150 arbitrary units (a.u.) and this is measured with a Sebumeter. As can be seen from Fig. 8, as the sebum level is varied from 0-150 arbitrary units (a.u.), the use of the existing device without a spacer provides a 2.5 times reduction in the skin hydration measurements even though the baseline hydration levels are constant, whereas this factor is reduced to less than 30% using the device 100 comprising the spacer 104 described herein. Thus, as illustrated in Fig. 8, the influence of the amount of lipids on the determined hydration level can be minimized using the device 100 comprising the spacer 104 described herein.

As mentioned earlier, the device 100 and method 300 described herein enables a lipid level of skin 200 to be determined more accurately. This is possible since the confounding influence of hydration (e.g. water or moisture) on the lipid (e.g. sebum) level measurements due to the different dielectric properties of hydration and lipids is minimized by the spacer 104 of the device 100 enabling the skin 200 response to be measured at different penetration depths for use in determining the lipid level of the skin 200. This can be observed by obtaining skin lipid measurements on a calibration pad.

In an example, skin lipid measurements were obtained using an existing device without a spacer, namely a Corneometer. The skin lipid measurements were obtained on a calibration pad mimicking a two-layer system. The calibration pad consisted of cellulose paper soaked with water of a known ionic content. The water concentration of the calibration pad was varied for different hydration levels (low, medium, high). On the top of the water layer, different amounts of sebum were applied to mimic different lipid levels or, more specifically, different oil conditions (low, medium, high). Measurements were performed with a Corneometer with and without spacer. The spacer used comprises a polyethylene terephthalate (PET) foil. The spacer had a thickness in a range from 0.5 to 0.9 µm. Similar results can also be obtained by using a finger print sensor, which provides large area mapping of skin properties. Other types of dielectric materials and thickness can also be used depending on the electrode geometry.

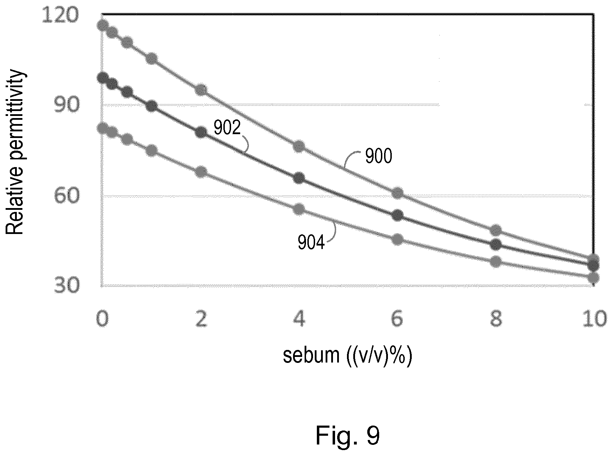

Fig. 9 illustrates the skin lipid measurements obtained using the existing device without the spacer, namely the Corneometer. In this example, the skin lipid measurements comprise relative permittivity measurements. The skin lipid measurements (on the y-axis) are illustrated for different hydration levels (illustrated by the different lines) and different lipid levels (on the x-axis). In this example, the hydration level comprises a water content and the lipid level comprises an amount of sebum. As the skin lipid measurements comprise relative permittivity measurements, the skin lipid measurements have no units and the amount of sebum is provided in percent concentration volume/volume ((v/v)%) in Fig. 9. The different hydration levels comprise a high water content 900, a medium water content 902 and a low water content 904. Fig. 9 thus illustrates the dependence of the skin lipid measurements on water and sebum content. In particular, as expected, the skin lipid measurements increase as the water content increases and, for a given water content, the skin lipid measurement drops as the amount of sebum increases.

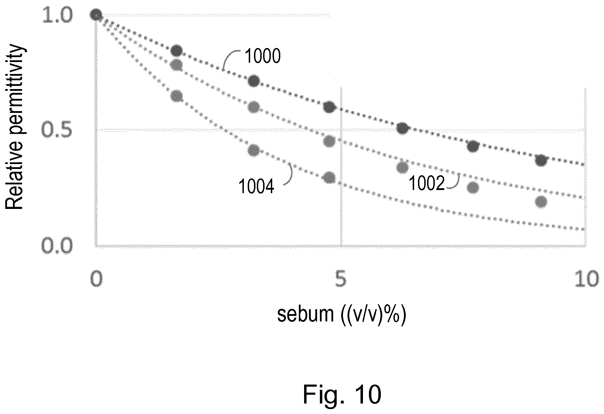

Fig. 10 illustrates the skin lipid measurements obtained using a skin response measured by a device 100 according to an embodiment. In this example, the skin lipid measurements comprise relative permittivity measurements. The skin lipid measurements (on the y-axis) are measured by the device 100 with no spacer and with spacers of different thicknesses (illustrated by the different lines) and different lipid levels (on the x-axis). In this example, the lipid level comprises an amount of sebum. As the skin lipid measurements comprise relative permittivity measurements, the skin lipid measurements have no units and the amount of sebum is provided in percent concentration volume/volume ((v/v)%) in Fig. 10. The different lines illustrate the skin lipid measurements as a function of lipid level with no spacer 1000, a spacer having a thickness of 0.5 µm 1002, and a spacer having a thickness of a 0.9 µm 1004. Thus, Fig. 10 shows the effect of confining the penetration (or probing) depth to superficial layers of skin 200 by using a spacer 104 in the manner described herein. As illustrated in Fig. 10, an increase of sensitivity to a sebum layer 202 occurs when the device 100 comprises a spacer 104 in the manner described herein. As also illustrated in Fig. 10, there is a further increase in sensitivity that occurs as the thickness of the spacer 104 is increased from 0.5 to 0.9 µm.

In some embodiments, the processor 116 described earlier can be configured to classify the determined hydration level and/or lipid level of the skin 200 into a class (or category). For example, the determined hydration level and/or the determined lipid level of the skin 200 may be classified into a type, such as a hydration and lipid (e.g. sebum or oiliness) type. The class (or category) into which the determined hydration level and/or lipid level of the skin 200 may be associated with a skin condition.

In some embodiments, the processor 116 may be configured to compare the response received from the skin 200 measured by the detector 110 described herein at the first penetration depth 106 (i.e. with the spacer 104) to the response received from the skin 200 measured by the detector 110 described herein at the second penetration depth 108 (i.e. without the spacer 104). In these embodiments, the processor 116 can be configured to classify the determined hydration level and/or the determined lipid level of the skin 200 into a class (or category) based on the comparison, e.g. based on the differences in the response received from the skin 200 measured by the detector 110 described herein at the first penetration depth 106 (i.e. with the spacer 104) and the response received from the skin 200 measured by the detector 110 described herein at the second penetration depth 108 (i.e. without the spacer 104).

That is, in some embodiments, the processor 116 can be configured to classify the determined hydration level and/or lipid level of the skin 200 into a class (or category) based on a comparison of the response received from the skin 200 at the different penetration depths 106, 108. In this way, changes in the response profile for the different penetration depths can be registered. As mentioned earlier, these responses may be measured permittivities according to some embodiments.

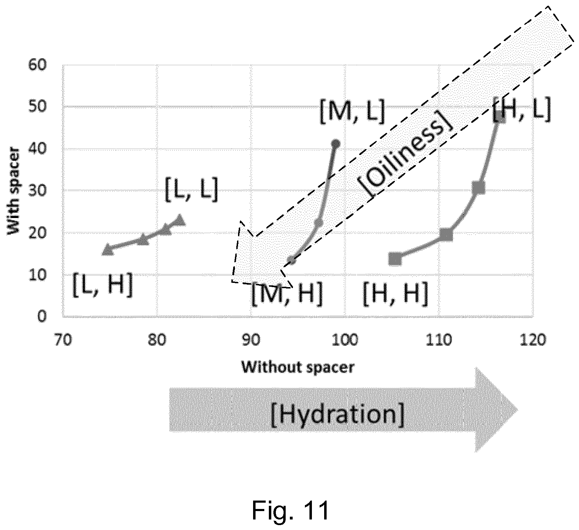

In some embodiments, as illustrated in Fig. 11, the response received from the skin 200 measured by the detector 110 described herein at the first penetration depth 106 (i.e. with the spacer 104) may be plotted as a function of the response received from the skin 200 measured by the detector 110 described herein at the second penetration depth 108 (i.e. without the spacer 104). In these embodiments, this plot can be used to classify hydration and lipid (e.g. oil) levels of skin into a class (or category). For example, the determined hydration level and/or the determined lipid level of the skin 200 may be classified into a type, such as a hydration and lipid (e.g. sebum or oiliness) type. As mentioned earlier, the responses received from the skin 200 can be a permittivity according to some embodiments.

In Fig. 11, the first and second letters in the brackets represent the amount of hydration and sebum respectively. L, M and H represents low, medium and high levels of each of these components based on a broad classification into three levels. The points in the top right corner of Fig. 11 correspond to a hydrated and low oily condition, whereas the points in the bottom right corner of Fig. 11 correspond to a hydrated and high oily condition. As the points move towards the left in Fig. 11, the amount of hydration drops. The type of representation illustrated in Fig. 11 may be referred to as a calibration plot. The calibration plot can be used to classify skin conditions into different skin hydration and lipid (e.g. sebum or oil) types. Thus, for example, a response received from the skin 200 measured by the detector 110 described herein at the first penetration depth 106 (i.e. with the spacer 104) and at the second penetration depth 108 (i.e. without the spacer 104) can be compared to a calibration plot, such as that illustrated in Fig. 11, to classify (e.g. identify) a skin condition, such as oily, oily-dry, dry, etc. The classification described herein may be performed in real-time in some embodiments.

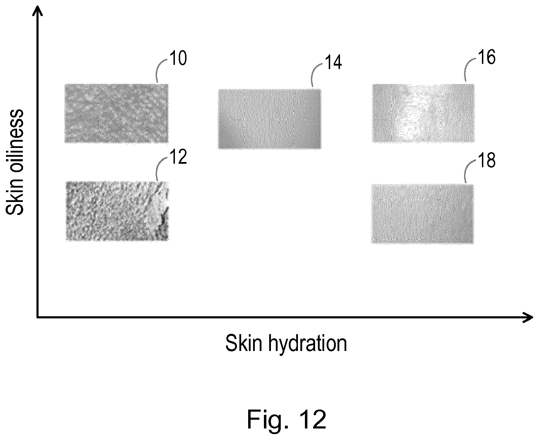

Fig. 12 illustrates a broad classification of different skin types based on the balance between a volume fraction of skin surface lipids ("Skin oiliness" on the y-axis) and a volume fraction of water ("Skin hydration" on the x-axis). In particular, as illustrated in Fig. 12, skin that is not hydrated and has excessive oil is classified as "dry and oily skin" 10, skin that is not hydrated and has low oiliness is classified as "dry skin" 12, skin that is sufficiently hydrated and has a sufficient oil level is classified as "normal skin" 14, skin that is hydrated and has excessive oil is classified as "hydrated and oily skin" 16, and skin that is hydrated and has low oiliness is classified as "hydrated skin" 18.

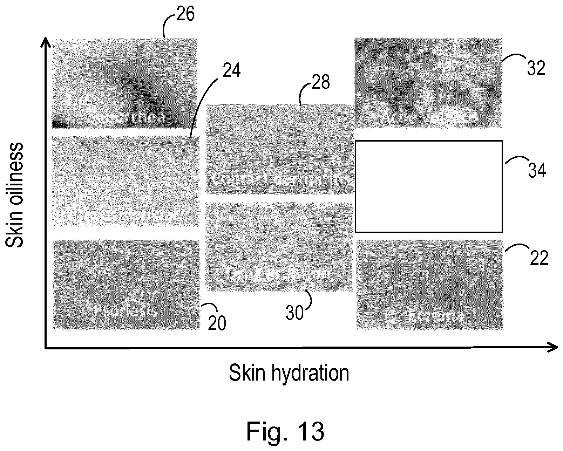

Skin hydration levels and lipid levels are considered to be important factors in skin health. In fact, as illustrated in Fig. 13, various skin disorders show peculiar skin conditions with respect to the balance between hydration and oiliness. For instance, skin conditions such atopic dermatitis shows drop in skin hydration level reflecting in a drop of water holding capacity of the skin, increased transepidermal water loss (TEWL) and a defect in barrier function. The same symptoms are seen in individuals suffering from psoriasis 20, eczema 22 and ichthyosis vulgaris 24. Eczema 22 leads to minor water loss (a few percent) combined with a noticeable oiliness drop (approximately 25%), whereas psoriasis 20 shows a dramatic decrease in hydration level (approximately 70%) and oiliness level (approximately 40-70%). Ichthyosis vulgaris 24 shows a decrease in hydration level (approximately 63%) while the level of superficial skin lipids does not vary significantly (approximately ±15%). Other skin conditions illustrated in Fig. 13 include seborrhea 26, contact dermatitis 28, drug eruption (e.g. allergy) 30, and acne vulgaris 32. Fig. 13 also illustrates the position of normal skin 34 with respect to the balance between hydration and oiliness, which has a sufficient hydration level and a sufficient oil level. Thus, a water-sebum system and its balance determines the condition of the skin and can be used as an indicator of skin health.

In some embodiments, the device 100 (e.g. a communications interface of the device 100) described herein may be configured to communicate information indicative of an identified skin condition to at least one other device, such as a mobile device (e.g. a phone, such as a smartphone, a tablet, etc.). The communication may, for example, be over a digital connection or any other connection. The connection may be wireless in some embodiments. The at least one other device may be configured to process the information indicative of an identified skin condition and generate a recommendation. The recommendation can be a recommendation regarding a skin regime. The recommendation may be rendered on a user interface (e.g. a screen or display) of the at least one other device.

In some embodiments (e.g. once a recommendation has been generated), the processor 116 of the device 100 may be configured to track the identified skin condition, e.g. to detect improvement or changes over time. In some embodiments, the identified skin condition may be tracked before and/or after a particular skin treatment or intervention, e.g. to determine the effect of that skin treatment or intervention. In some embodiments, the skin condition can be used (e.g. in real time) during treatment of the skin 200. In some embodiments, the skin condition may be quantitatively measured before and after the treatment by taking into account the variation in hydration and lipid levels of the skin at different locations and under different climatic variations.

There is thus provided herein an improved device 100 and method 300 of operating the device 100 for determining a hydration level and/or lipid level of skin 200. The device 100 can be portable. The device 100 is low cost, fast and easy to use. The device 100 makes it possible to non-invasively measure a hydration level and/or a lipid level of skin (e.g. and even simultaneously measure the hydration level and lipid level of skin) in view of the manner in which the spacer is arranged to allow different penetration depths for measurements. The storage of measured data, such as the measured hydration level and/or a lipid level of skin, enables skin conditions to be monitored and controlled of over time. The possibility of measuring both a hydration level and a lipid level of skin (e.g. simultaneously) enables the balance between these two factors, which are related to skin health, to be assessed and also enables selection of an appropriate skin care treatment and products.

There is also provided a computer program product comprising a non-transitory computer readable medium, the computer readable medium having a computer readable code embodied therein, the computer readable code being configured such that, on execution by a suitable computer or processor, the computer or processor is caused to perform the method described herein.

It will thus be appreciated that the embodiments described herein also apply to computer programs, particularly computer programs on or in a carrier, adapted to put the disclosure into practice. The program may be in the form of a source code, an object code, a code intermediate source and an object code such as in a partially compiled form, or in any other form suitable for use in the implementation of the method described herein. It will also be appreciated that such a program may have many different architectural designs. For example, a program code implementing the functionality of the method or device described herein may be sub-divided into one or more sub-routines. A variety of different ways of distributing the functionality among these sub-routines will be apparent to a person skilled in the art. The sub-routines may be stored together in one executable file to form a self-contained program. Such an executable file may comprise computer-executable instructions, for example, processor instructions and/or interpreter instructions (e.g. Java interpreter instructions).

Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of the device set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the method described herein.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A device (100) for use in determining a hydration level and/or lipid level of skin (200), the device (100) comprising:
a plurality of electrodes (102) configured to provide an electrical signal to the skin (200);
a spacer (104) arranged to be positioned between at least one of the plurality of electrodes (102) and the skin (200) when the device (100) is in use, such that the at least one of the plurality of electrodes (102) and at least another one of the plurality electrodes (102) are configured to provide the electrical signal to the skin (200) at different penetration depths (106, 108); and
a detector (110) configured to measure a response received from the skin (200) at the different penetration depths (106, 108) for use in determining the hydration level and/or lipid level of the skin (200).

2. The device (100) as claimed in claim 1, wherein:
the spacer (104) is arranged to be positioned between at least one of the plurality of electrodes (102) and the skin (200) when the device (100) is in use, such that:
the at least one of the plurality of electrodes (102) is configured to provide the electrical signal to the skin (200) at a first penetration depth (106) from the at least one of the plurality of electrodes (102) to a surface of the skin (200); and
the at least another one of the plurality electrodes (102) is configured to provide the electrical signal to the skin (200) at a second penetration depth (108) from the at least another one of the plurality of electrodes (102) to a location beneath the surface of the skin (200).

3. The device (100) as claimed in claim 1 or 2, wherein the spacer (104) comprises a dielectric spacer.

4. The device (100) as claimed in any of the preceding claims, wherein the spacer (104) is patterned such that the at least one of the plurality of electrodes and the at least another one of the plurality electrodes (102) provide the electrical signal to the skin (200) at the different penetration depths (106, 108).

5. The device (100) as claimed in any of the preceding claims, wherein a composition and/or thickness of the spacer (104) is selected based on a geometry of the at least one of the plurality of electrodes (102) and the at least another one of the plurality electrodes (102).

6. The device (100) as claimed in any of the preceding claims, wherein the spacer (104) comprises a polyethylene terephthalate material, a polyelectric material, an aluminium material, or a polyetheretherketone material.

7. The device (100) as claimed in any of the preceding claims, wherein the spacer (104) is of a predefined thickness in a range from 1 micron to 100 microns.

8. The device (100) as claimed in any of the preceding claims, wherein the plurality of electrodes (102) comprise at least two pixelated electrodes.

9. The device (100) as claimed in any of the preceding claims, the device (100) further comprising:
a signal generator (112) configured to:
generate the electrical signal; and
provide the electrical signal to the plurality of electrodes (102).

10. A system for determining a hydration level and/or lipid level of skin (200), the system comprising:
a device (100) as claimed in any of the preceding claims; and
a processor (116) configured to:
acquire, from the detector (110), the measured response received from the skin (200) at the different penetration depths (106, 108); and
determine the hydration level and/or lipid level of the skin (200) based on the measured response received from the skin (200) at the different penetration depths (106, 108).

11. A system as claimed in claim 10, wherein:
the processor (116) is configured to:
classify the determined hydration level and/or lipid level of the skin (200) into a class based on a comparison of the response received from the skin 200 at the different penetration depths (106, 108).

12. A method of operating a device (100) for use in determining a hydration level and/or lipid level of skin (200), the device (100) comprising a plurality of electrodes (102), a spacer (104), and a detector (110), wherein the method comprises:
providing, by the plurality of electrodes (102), an electrical signal to the skin (200), wherein the spacer (104) is arranged to be positioned between at least one of the plurality of electrodes (102) and the skin (200) when the device (100) is in use, such that the at least one of the plurality of electrodes (102) and at least another one of the plurality electrodes (102) provide the electrical signal to the skin (200) at different penetration depths (106, 108); and
measuring, by the detector (110), a response received from the skin (200) at the different penetration depths (106, 108) for use in determining the hydration level and/or lipid level of the skin (200).

13. The method as claimed in claim 12, the method further comprising:
generating, by a signal generator (112), the electrical signal; and
providing, by the signal generator (112), the electrical signal to the plurality of electrodes (102).

14. The method as claimed in claim 12 or 13, wherein the method further comprises:
acquiring, by a processor (116) from the detector (110), the measured response received from the skin (200) at the different penetration depths (106, 108); and
determining, by the processor (116), the hydration level and/or lipid level of the skin (200) based on the measured response received from the skin (200) at the different penetration depths (106, 108).

15. A computer program product comprising a non-transitory computer readable medium, the computer readable medium having a computer readable code embodied therein, the computer readable code being configured such that, on execution by a suitable computer or processor, the computer or processor is caused to perform the method as claimed in any of claims 12 to 14.

## Patentansprüche

1. Vorrichtung (100) zur Verwendung bei der Bestimmung eines Hydratations- und/oder Lipidniveaus der Haut (200), wobei die Vorrichtung (100) umfasst: eine Vielzahl von Elektroden (102), die so konfiguriert sind, dass sie ein elektrisches Signal an die Haut (200) liefern; einen Abstandshalter (104), der so angeordnet ist, dass er zwischen mindestens einer der mehreren Elektroden (102) und der Haut (200) positioniert wird, wenn die Vorrichtung (100) in Gebrauch ist, so dass die mindestens eine der mehreren Elektroden (102) und mindestens eine andere der mehreren Elektroden (102) so konfiguriert sind, dass sie das elektrische Signal an die Haut (200) mit unterschiedlichen Eindringtiefen (106, 108) liefern; und einen Detektor (110), der so konfiguriert ist, dass er eine von der Haut (200) bei den verschiedenen Eindringtiefen (106, 108) empfangene Reaktion misst, um den Hydratationsgrad und/oder den Lipidgehalt der Haut (200) zu bestimmen.

2. Die Vorrichtung (100) nach Anspruch 1, wobei: der Abstandshalter (104) so angeordnet ist, dass er zwischen mindestens einer der Vielzahl von Elektroden (102) und der Haut (200) positioniert ist, wenn die Vorrichtung (100) in Gebrauch ist, so dass: mindestens eine der Vielzahl von Elektroden (102) so konfiguriert ist, so dass das elektrischen Signal der Haut (200) in einer ersten Eindringtiefe (106) aus mindestens eine der mehreren Elektroden (102) an einer Oberfläche der Haut (200) anbringt; und mindestens eine andere der mehreren Elektroden (102) so konfiguriert ist, dass das elektrischen Signal der Haut (200) in einer zweiten Eindringtiefe (108) von mindestens einer anderen der mehreren Elektroden (102) zu einer Stelle unterhalb der Oberfläche der Haut (200) liefern.

3. Die Vorrichtung (100) nach Anspruch 1 oder 2, wobei der Abstandshalter (104) einen dielektrischen Abstandshalter umfasst.

4. Vorrichtung (100) nach einem der vorhergehenden Ansprüche, wobei der Abstandshalter (104) so gestaltet ist, dass mindestens eine der mehreren Elektroden und mindestens eine andere der Vielzahl von Elektroden (102) das elektrische Signal an die Haut (200) in den unterschiedlichen Eindringtiefen (106, 108) liefern.

5. Vorrichtung (100) nach einem der vorhergehenden Ansprüche, wobei eine Zusammensetzung und/oder Dicke des Abstandshalters (104) auf der Grundlage einer Geometrie mindestens einer der Vielzahl von Elektroden (102) und mindestens einer anderen der Vielzahl von Elektroden (102) ausgewählt wird.

6. Vorrichtung (100) nach einem der vorhergehenden Ansprüche, wobei der Abstandshalter (104) Polyethylenterephthalat, polyelektrisches Material, Aluminiummaterial oder Polyetheretherketon umfasst.

7. Die Vorrichtung (100) nach einem der vorhergehenden Ansprüche, wobei der Abstandshalter (104) eine vordefinierte Dicke in einem Bereich von 1 Mikron bis 100 Mikron aufweist.

8. Vorrichtung (100) nach einem der vorangehenden Ansprüche, wobei die Mehrzahl der Elektroden (102) mindestens zwei gepixelte Elektroden umfasst.

9. Die Vorrichtung (100) nach einem der vorhergehenden Ansprüche, wobei die Vorrichtung (100) weiterhin umfasst: einen Signalgenerator (112), der so konfiguriert ist, dass er: das elektrische Signal erzeugt; und das elektrische Signal an die Vielzahl von Elektroden (102) liefert.

10. System zur Bestimmung des Hydratationsgrades und/oder des Lipidgrades der Haut (200), wobei das System umfasst: eine Vorrichtung (100) nach einem der vorangehenden Ansprüche; und einen Prozessor (116), der dafür konfiguriert ist: von dem Detektor (110) die gemessene Antwort der Haut (200) in den verschiedenen Eindringtiefen (106, 108) zu erfassen; und das Hydratations- und/oder Lipidniveaus der Haut (200) auf der Grundlage der gemessenen Reaktion zu bestimmen, die von der Haut (200) bei den verschiedenen Eindringtiefen (106, 108) empfangen wurde.

11. System nach Anspruch 10, wobei: der Prozessor (116) konfiguriert ist, um: den ermittelten Hydratationsgrad und/oder Lipidgehalt der Haut (200) auf der Grundlage eines Vergleichs der von der Haut (200) bei den verschiedenen Eindringtiefen (106, 108) erhaltenen Antwort in eine Klasse einzuordnen.

12. Verfahren zum Betrieb einer Vorrichtung (100) zur Bestimmung des Hydratations- und/oder Lipidsniveaus der Haut (200), wobei die Vorrichtung (100) eine Vielzahl von Elektroden (102), einen Abstandshalter (104) und einen Detektor (110) umfasst, wobei das Verfahren umfasst: bereitstellung eines elektrischen Signals für die Haut durch die Vielzahl von Elektroden (102) ein elektrisches Signal an die Haut (200), wobei der Abstandshalter (104) so angeordnet ist, dass er sich zwischen mindestens einem der mehrere Elektroden (102) und die Haut (200), wenn die Vorrichtung (100) in Gebrauch ist, so dass mindestens eine der Vielzahl von Elektroden (102) und mindestens eine andere der Vielzahl von Elektroden (102) das elektrische Signal an die Haut (200) in unterschiedlichen Eindringtiefen (106, 108) liefern; und messen einer von der Haut (200) bei den verschiedenen Eindringtiefen (106, 108) empfangenen Antwort durch den Detektor (110) zur Verwendung bei der Bestimmung des Hydratations- und/oder des Lipidniveaus der Haut (200).

13. Verfahren nach Anspruch 12, wobei das Verfahren ferner umfasst: erzeugung des elektrischen Signals durch einen Signalgenerator (112); und bereitstellung des elektrischen Signals durch den Signalgenerator (112) für die Mehrzahl der Elektroden (102).

14. Verfahren nach Anspruch 12 oder 13, wobei das Verfahren ferner umfasst: Erfassung der gemessenen Antwort, die von der Haut (200) bei den verschiedenen Eindringtiefen (106, 108) empfangen wurde, durch einen Prozessor (116) aus dem Detektor (110); und bestimmung, durch den Prozessor (116), des Hydratations- und/oder Lipidniveaus der Haut (200), basierend auf der gemessenen Antwort, die von der Haut (200) bei den verschiedenen Eindringtiefen (106, 108) empfangen wurde.

15. Ein Computerprogrammprodukt, das ein nichttransitorisches computerlesbares Medium umfasst, wobei das computerlesbare Medium einen darin verkörperten computerlesbaren Code aufweist, der computerlesbare Code so konfiguriert ist, dass bei Ausführung durch einen geeigneten Computer oder Prozessor der Computer oder Prozessor veranlasst wird, das Verfahren nach einem der folgenden Ansprüche 12 bis 14 durchzuführen.

## Revendications

1. Dispositif (100) utilisé pour déterminer le niveau d'hydratation et/ou le niveau de lipides de la peau (200), le dispositif (100) comprenant: une pluralité d'électrodes (102) configurées pour fournir un signal électrique à la peau (200); une entretoise (104) conçue pour être positionnée entre au moins une des électrodes (102) et la peau (200) lorsque le dispositif (100) est utilisé, de sorte qu'au moins une des électrodes (102) et au moins une autre des électrodes (102) sont configurées pour fournir le signal électrique à la peau (200) à différentes profondeurs de pénétration (106, 108); et un détecteur (110) configuré pour mesurer une réponse reçue de la peau (200) aux différentes profondeurs de pénétration (106, 108) afin de déterminer le niveau d'hydratation et/ou le niveau de lipides de la peau (200) .

2. Dispositif (100) selon la revendication 1, dans lequel: l'entretoise (104) est agencée pour être positionnée entre au moins une des électrodes (102) et la peau (200) lorsque le dispositif (100) est en cours d'utilisation, de telle sorte que: l'une au moins de la pluralité d'électrodes (102) est configurée pour fournir le signal électrique à la peau (200) à une première profondeur de pénétration (106) depuis l'une au moins de la pluralité d'électrodes (102) jusqu'à une surface de la peau (200); et au moins une autre électrode de la pluralité (102) est configurée pour fournir le signal électrique à la peau (200) à une deuxième profondeur de pénétration (108) depuis au moins une autre électrode de la pluralité (102) jusqu'à un endroit situé sous la surface de la peau (200).

3. Dispositif (100) selon la revendication 1 ou 2, dans lequel l'entretoise (104) comprend une entretoise diélectrique.

4. Dispositif (100) selon l'une des revendications précédentes, dans lequel l'entretoise (104) est conçue de manière que l'une au moins de la pluralité d'électrodes et l'autre au moins de la pluralité d'électrodes (102) fournissent le signal électrique à la peau (200) aux différentes profondeurs de pénétration (106, 108).

5. Dispositif (100) selon l'une quelconque des revendications précédentes, dans lequel une composition et/ou une épaisseur de l'entretoise (104) est sélectionnée en fonction d'une géométrie de l'une au moins de la pluralité d'électrodes (102) et de l'autre au moins de la pluralité d'électrodes (102).

6. Dispositif (100) selon l'une quelconque des revendications précédentes, dans lequel l'entretoise (104) comprend un matériau en polyéthylène téréphtalate, un matériau polyélectrique, un matériau en aluminium ou un matériau en polyétheréthercétone.

7. Dispositif (100) selon l'une quelconque des revendications précédentes, dans lequel l'entretoise (104) a une épaisseur prédéfinie comprise entre 1 micron et 100 microns.

8. Dispositif (100) selon l'une des revendications précédentes, dans lequel la pluralité d'électrodes (102) comprend au moins deux électrodes pixellisées.

9. Dispositif (100) selon l'une quelconque des revendications précédentes, le dispositif (100) comprenant en outre: un générateur de signal (112) configuré pour: générer le signal électrique; et fournir le signal électrique à la pluralité d'électrodes (102).

10. Système de détermination d'un niveau d'hydratation et/ou d'un niveau de lipides de la peau (200), le système comprenant: un dispositif (100) tel que revendiqué dans l'une quelconque des revendications précédentes; et un processeur (116) configuré pour: acquérir, à partir du détecteur (110), la réponse mesurée reçue de la peau (200) aux différentes profondeurs de pénétration (106, 108); et déterminer le niveau d'hydratation et/ou le niveau de lipides de la peau (200) sur la base de la réponse mesurée reçue de la peau (200) aux différentes profondeurs de pénétration (106, 108).

11. Système selon la revendication 10, dans lequel: le processeur (116) est configuré pour: classer le niveau d'hydratation déterminé et/ou le niveau de lipides de la peau (200) dans une catégorie sur la base d'une comparaison de la réponse reçue de la peau 200 aux différentes profondeurs de pénétration (106, 108) .

12. Méthode d'utilisation d'un dispositif (100) pour déterminer le niveau d'hydratation et/ou le niveau de lipides de la peau (200), le dispositif (100) comprenant une pluralité d'électrodes (102), une entretoise (104), et un détecteur (110), dans laquelle la méthode comprend: fournir, par la pluralité d'électrodes (102), un signal électrique à la peau (200), dans lequel l'entretoise (104) est conçue pour être positionnée entre au moins une de la pluralité d'électrodes (102) et la peau (200) lorsque le dispositif (100) est utilisé, de telle sorte qu'au moins une de la pluralité d'électrodes (102) et au moins une autre de la pluralité d'électrodes (102) fournissent le signal électrique à la peau (200) à différentes profondeurs de pénétration (106, 108); et mesurer, par le détecteur (110), une réponse reçue de la peau (200) aux différentes profondeurs de pénétration (106, 108) afin de déterminer le niveau d'hydratation et/ou le niveau de lipides de la peau (200) .

13. Méthode selon la revendication 12, la méthode comprenant en outre: générer, par un générateur de signaux (112), le signal électrique; et fournir, par le générateur de signaux (112), le signal électrique à la pluralité d'électrodes (102).

14. Méthode selon la revendication 12 ou 13, dans laquelle la méthode comprend en outre: l'acquisition, par un processeur (116) du détecteur (110), de la réponse mesurée reçue de la peau (200) aux différentes profondeurs de pénétration (106, 108); et déterminer, par le processeur (116), le niveau d'hydratation et/ou le niveau de lipides de la peau (200) sur la base de la réponse mesurée reçue de la peau (200) aux différentes profondeurs de pénétration (106, 108).

15. Produit de programme informatique comprenant un support lisible par ordinateur non transitoire, le support lisible par ordinateur ayant un code lisible par ordinateur incorporé, le code lisible par ordinateur est configuré de telle sorte que, lors de l'exécution par un ordinateur ou un processeur approprié, l'ordinateur ou le processeur est amené à exécuter la méthode telle que revendiquée dans l'une des revendications 12 à 14.
